(19) Europäisches Patentamt
European Patent Office
Office européen des brevets

(11) **EP 4 520 277 A1**

(12) **EUROPEAN PATENT APPLICATION**
published in accordance with Art. 153(4) EPC

(43) Date of publication:
**12.03.2025 Bulletin 2025/11**

(21) Application number: **22940743.2**

(22) Date of filing: **12.10.2022**

(51) International Patent Classification (IPC):
**A61B 17/32** (2006.01)          **B06B 3/00** (2006.01)

(86) International application number:
**PCT/CN2022/124733**

(87) International publication number:
**WO 2023/213049 (09.11.2023 Gazette 2023/45)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **05.05.2022   CN 202210482267**

(71) Applicant: **Innolcon Medical Technology (Suzhou)
Co., Ltd.
Jiangsu 215000 (CN)**

(72) Inventors:
• WANG, Haiyang
  Suzhou, Jiangsu 215000 (CN)
• WANG, Lei
  Suzhou, Jiangsu 215000 (CN)
• LUO, Wei
  Suzhou, Jiangsu 215000 (CN)

(74) Representative: **AWA Sweden AB
Matrosgatan 1
Box 5117
200 71 Malmö (SE)**

(54) **FREQUENCY TRACKING METHOD AND SYSTEM FOR ULTRASONIC TRANSDUCER AND ULTRASONIC DEVICE**

(57)    A frequency tracking method and system for ultrasonic transducer and an ultrasonic device are disclosed. The frequency tracking method for ultrasonic transducer includes the following steps: S1, calculating the phase difference of voltage and current based on acquired voltage and current signals; S2, determining whether the phase difference of the voltage and current is greater than a target phase difference; S3, if not, reducing the operating frequency and calculating the adjusted operating frequency; S4, if yes, calculating a phase difference change rate, determining whether to increase or decrease the operating frequency based on the phase difference change rate, and calculating the adjusted operating frequency. When performing ultrasonic transducer frequency tracking, the method uses the phase difference change rate to distinguish the working state of the ultrasonic transducer, thereby avoiding the problem that frequency locking fails because the minimum value of the phase difference of voltage and current corresponding to the working frequency range is greater than the phase-locking phase, and that the ultrasonic device is unable to work normally. At the same time, automatic frequency tracking is effectively realized, and the working efficiency and stability of the ultrasonic device are improved.

FIG 3

EP 4 520 277 A1

**Description**

**FIELD OF THE INVENTION**

The present disclosure relates to the field of ultrasonic devices, in particular to a frequency tracking method and system for ultrasonic transducer and an ultrasonic device.

**BACKGROUND OF THE INVENTION**

The ultrasonic transducer is an important part of various ultrasonic devices, which plays an indispensable role especially in ultrasonic medical devices. When the ultrasonic device is working, the output frequency of the ultrasonic power supply and the resonant frequency of the ultrasonic transducer must be consistent or close to each other, so that the ultrasonic transducer can work with the highest efficiency. However, the ultrasonic transducer will age as the temperature and environment change during the production or use process, and its resonant frequency will shift. Therefore, it is necessary to track and lock the resonant frequency of the ultrasonic transducer to ensure that the ultrasonic transducer can work at the appropriate operating frequency.

The Chinese patent application No. 201810159303.6 discloses an operating frequency control method of an ultrasonic surgical tool, wherein it discloses a method for real-time tracking of the operating frequency after performing corresponding calculations by detecting the phase difference of voltage and current.

In another method of real-time tracking of the operating frequency by detecting the phase difference of voltage and current, the tracking formula used is as follows:

$$f_{n+1} = f_n + \Delta f \ .$$

In the formula, $f_{n+1}$ represents the working frequency after adjustment, $f_n$ represents the working frequency before adjustment, and $\Delta f$ represents the frequency adjustment amount, which can be calculated according to the following formula:

$$\Delta f = K \Delta \theta$$

In the formula, $K$ is the variable frequency coefficient, taking positive values; $\Delta \theta$ is the difference between the phase difference of voltage and current and the target phase difference. The target phase difference can be obtained by reading, for example, the parameters of the ultrasonic handle.

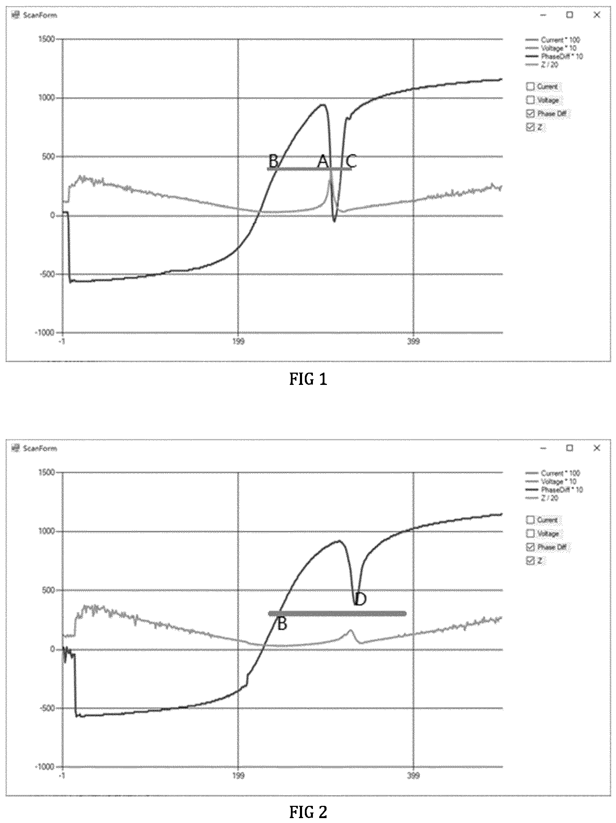

The ultrasonic transducer is a narrowband system, as shown in FIG 1. Assuming that the target phase difference is point A, and the phase difference of voltage and current corresponding to the working frequency of the ultrasonic transducer passes through point A, then the above tracking algorithm can work normally in the range from point B to point C.

However, since the phase difference of voltage and current will change with load, etc., the minimum phase difference of voltage and current corresponding to the working frequency of the ultrasonic transducer may be greater than the target phase difference. As shown in FIG 2, there is no target phase difference A. In this circumstance, when the working frequency is calculated according to the above tracking formula, the working frequency will continue to increase, resulting in frequency locking failure. Therefore, a method is needed to avoid this problem.

**SUMMARY OF THE INVENTION**

The purpose of the present disclosure is to solve the above problems existing in the prior art and to provide a frequency tracking method and system for ultrasonic transducer and an ultrasonic device.

The purpose of the present disclosure is achieved through the following solutions.

A frequency tracking method for ultrasonic transducer is provided, which includes the following steps:

S1, calculating a phase difference of voltage and current according to acquired voltage and current signals;
S2, determining whether the phase difference of voltage and current is greater than a target phase difference;
S3, reducing the operating frequency and calculating the adjusted operating frequency when it is determined that the phase difference of voltage and current is not greater than the target phase difference;
S4, calculating a phase difference change rate, determining whether to increase or decrease the operating frequency according to the phase difference change rate, and calculating the adjusted operating frequency, when it is determined that the phase difference of voltage and current is greater than the target phase difference.

Preferably, the phase difference change rate is calculated according to formula (1) as follows:

$$R_P = (P_n - P_{n-1}) \div (f_n - f_{n-1}) \qquad (1).$$

In the formula, $R_P$ is the phase difference change rate; $P_n$ is the phase difference of voltage and current calculated this time; $P_{n-1}$ is the phase difference of voltage and current calculated last time; $f_n$ is the operating frequency corresponding to the phase difference of voltage and current calculated this time; and $f_{n-1}$ is the operating frequency corresponding to the phase difference of voltage and current calculated last time.

Preferably, in step S3, when calculating the adjusted operating frequency, the calculation is performed according to formula (2) as follows:

$$f_{n+1} = f_n + \frac{k}{z}\Delta\theta \qquad (2).$$

In the formula, $f_{n+1}$ is the operating frequency after adjustment; $f_n$ is the operating frequency before adjustment; z is the impedance; k is a coefficient factor; and $\Delta\theta$ is the difference between the phase difference of voltage and current and the target phase difference.

Preferably, in S4, determining whether to increase or decrease the operating frequency according to the phase difference change rate, and calculating the adjusted operating frequency includes:

S41, determining whether the phase difference change rate is less than zero;
S42, increasing the operating frequency and calculating the adjusted operating frequency when it is determined that the phase difference change rate is less than zero;
S43, determining whether the phase difference change rate is within a preset range when it is determined that the phase difference change rate is not less than zero;
S44, increasing the operating frequency and calculating the adjusted operating frequency when it is determined that the phase difference change rate is within the preset range; and
S45, reducing the operating frequency and calculating the adjusted operating frequency when it is determined that the phase difference change rate is not within the preset range.

Preferably, the preset range is $K_\theta \pm \varepsilon$, wherein $K_\theta$ can be obtained by calculating the slope value of an experimental sweep frequency curve, and $\varepsilon$ is a set adjustment margin.

Preferably, in S42 and/or S44, when calculating the adjusted operating frequency, the calculation is performed according to formula (3) as follows:

$$f_{n+1} = f_n + \frac{l}{\omega}\Delta\theta \qquad (3).$$

In the formula, $f_{n+1}$ is the operating frequency after adjustment; $f_n$ is the operating frequency before adjustment; $\omega$ is an absolute value of the phase difference change rate; $l$ is a proportionality factor; and $\Delta\theta$ is the difference between the phase difference of voltage and current and the target phase difference.

Preferably, in S45, when calculating the adjusted operating frequency, the calculation is performed according to formula (4) as follows:

$$f_{n+1} = f_n - \frac{k}{z}\Delta\theta \qquad (4).$$

In the formula, $f_{n+1}$ is the operating frequency after adjustment; $f_n$ is the operating frequency before adjustment; z is the impedance; k is a coefficient factor; and $\Delta\theta$ is the difference between the phase difference of voltage and current and the target phase difference.

Preferably, in S45, when calculating the adjusted operating frequency, the calculation is performed according to formula (5) as follows:

$$f_{n+1} = f_n - \frac{l}{\omega}\Delta\theta \qquad (5).$$

In the formula, $f_{n+1}$ is the operating frequency after adjustment; $f_n$ is the operating frequency before adjustment; $\omega$ is the absolute value of the phase difference change rate; $l$ is the proportionality factor; and $\Delta\theta$ is the difference between the phase difference of voltage and current and the target phase difference.

An operating frequency tracking system for ultrasonic transducer, which includes:

A phase difference determination unit, configured to calculate a phase difference of voltage and current according to acquired voltage and current signals;

A judgment unit, configured to determine whether the phase difference of voltage and the current is greater than a target phase difference;

A first adjustment unit, configured to reduce the operating frequency and calculate the adjusted operating frequency when the phase difference of voltage and current is not greater than the target phase difference; and

A second adjustment unit, configured to calculate a phase difference change rate, determine whether to increase or decrease the operating frequency according to the phase difference change rate, and calculate the adjusted operating frequency, when the phase difference of voltage and the current is greater than the target phase difference.

An ultrasound device is provided, which includes a processor and a memory, wherein the memory stores a program that can be called by the processor, and when the processor executes the program, any tracking method as described above is implemented.

The advantages of the solution of the present disclosure are mainly reflected in the following.

When tracking the frequency of the ultrasonic transducer, the solution distinguishes the working state of the ultrasonic transducer in combination with the phase difference change rate, thereby avoiding the problem that frequency locking fails and the ultrasonic device to fail to work normally caused by the fact that the minimum value of the phase difference of voltage and current corresponding to the working frequency range is greater than the frequency locking phase. At the same time, automatic frequency tracking is effectively realized, improving the working efficiency and stability of the ultrasonic device.

When calculating the adjusted operating frequency, the solution performs calculation based on the phase difference change rate, which can make the calculation result more accurate, thereby improving the tracking accuracy.

**BRIEF DESCRIPTION OF THE DRAWINGS**

FIG 1 is a frequency/phase-difference/impedance diagram with a target phase difference described in the background of the present disclosure (the dark black line in the figure is a phase difference variation curve).

FIG 2 is a frequency/phase-difference/impedance diagram without a target phase difference described in the background of the present disclosure (the dark black line in the figure is a phase difference variation curve).

FIG 3 is a process diagram of the tracking method according to the present disclosure.

FIG 4 is a flow chart of the tracking method according to the present disclosure.

**DETAILED DESCRIPTION OF THE EMBODIMENTS**

The purpose, advantages and features of the present disclosure will be illustrated and explained by the non-limiting description of the following preferred embodiments. These embodiments are only typical examples of the application of the solution of the present disclosure, and any technical solution formed by equivalent replacement or equivalent transformation falls within the scope claimed by the present disclosure.

In the description of the solution, it should be noted that the terms "center", "up", "down", "left", "right", "front", "back", "vertical", "horizontal", "inside", "outside" and the like indicate positions or positional relationships based on the positions or positional relationships shown in the drawings, which are only for the convenience and simplification of description, and do not indicate or imply that the device or element referred to must have a specific orientation, be constructed and operated in a specific orientation, and therefore cannot be understood as a limitation of the present disclosure. In addition, the terms "first", "second", and "third" are used for descriptive purposes only, and cannot be understood as indicating or implying relative importance. Moreover, in the description of the solution, with the operator as a reference, the direction close to the operator is the proximal end, and the direction away from the operator is the distal end.

Example 1

The frequency tracking method for ultrasonic transducer disclosed in the present disclosure is described below in conjunction with the accompanying drawings. When tracking the frequency of the ultrasonic transducer, the solution distinguishes the working state of the ultrasonic transducer in combination with the phase difference change rate, thereby avoiding the problem that frequency locking fails and the ultrasonic device to fail to work normally caused by the fact that the minimum value of the phase difference of voltage and current corresponding to the working frequency range of the ultrasonic generator is greater than the target phase difference (frequency locking phase), and automatic frequency tracking is effectively realized, improving the working efficiency and stability of the ultrasonic device.

The above improvements are made because the inventors found through testing that when the ultrasonic transducer is working, the phase difference change rate has a certain regularity. When analyzing, in combination with FIG 1 and FIG 2, the phase difference of voltage and current before point B is not discussed, and the corresponding phase difference change regularity is shown in Tables 1 and 2.

Table 1: Relationship between phase difference change rate and frequency adjustment in FIG 1

| Range | Phase Difference | Phase Difference Change Rate | Frequency Adjustment |
|---|---|---|---|
| Rising range from point B to point A | Phase difference is greater than target phase difference | Phase difference change rate is positive and the magnitude is basically constant and is not related to load change | Increasing frequency to track phase difference |
| Descent range from point B to point A | Phase difference is greater than target phase difference | Phase difference change rate is negative | Increasing frequency to track phase difference |
| Descent range from point A to point C | Phase difference is smaller than target phase difference | Phase difference change rate is negative | Reducing frequency to track phase difference |
| Rising range from point A to point C | Phase difference is smaller than target phase difference | Phase difference change rate is positive | Reducing frequency to track phase difference |
| Range after point C | Phase difference is greater than target phase difference | Phase difference change rate is positive and the magnitude is not determined to be related to load change | Reducing frequency to track phase difference |

Table 2: Relationship between phase difference change rate and frequency adjustment in FIG 2

| Range | Phase Difference | Phase Difference Change Rate | Frequency Adjustment |
|---|---|---|---|
| Rising range from point B to point D | Phase difference is greater than target phase difference | Phase difference change rate is positive and the magnitude is basically constant and is not related to load change | Increasing frequency to track phase difference |
| Descent range from point B to point D | Phase difference is greater than target phase difference | Phase difference change rate is negative | Increasing frequency to track phase difference |
| Range after point D | Phase difference is greater than target phase difference | Phase difference change rate is positive and the magnitude is not determined to be related to load change | Reducing frequency to track phase difference |

From Table 1 and Table 2 above, it can be determined that if the phase difference change rate is within a certain range, it is still in the effective area of the frequency tracking algorithm, and the frequency tracking formula can still be used for frequency tracking. Otherwise, it is necessary to invert the phase difference $\Delta f$. Therefore, when tracking the frequency, the phase difference change rate can be calculated to distinguish the different ranges where the voltage and current phase differences are greater than the target phase difference, thereby determining the adjustment direction of the operating frequency.

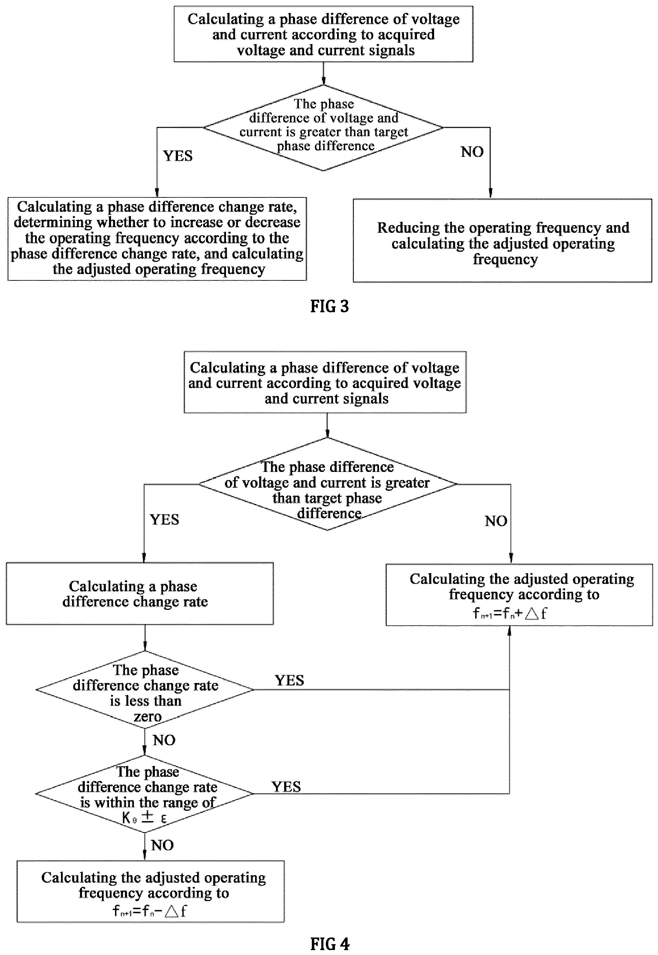

Specifically, as shown in FIG 3, the tracking method includes the following steps:

S1, calculating the phase difference of voltage and current according to acquired voltage and current signals;

S2, determining whether the phase difference of the voltage and the current is greater than a target phase difference;

S3, if not, that is, it is determined that the phase difference of voltage and current is not greater than the target phase difference, then reducing the operating frequency and calculating the adjusted operating frequency;

S4: If yes, that is, it is determined that the phase difference of voltage and current is greater than the target phase difference, then calculating the phase difference change rate, determining whether to increase or decrease the operating frequency according to the phase difference change rate, and calculating the adjusted operating frequency.

The phase difference change rate is calculated according to the formula (1) as follows:

$$R_P = (P_n - P_{n-1}) \div (f_n - f_{n-1}) \qquad (1).$$

In the formula, $R_P$ is the phase difference change rate; $P_n$ is the phase difference of voltage and current calculated this time; $P_{n-1}$ is the phase difference of voltage and current calculated last time; $f_n$ is the operating frequency corresponding to the phase difference of voltage and current calculated this time; and $f_{n-1}$ is the operating frequency corresponding to the phase difference of voltage and current calculated last time.

As shown in FIG 4, in S3, when calculating the adjusted operating frequency, the calculation is performed according to formula (2) as follows:

$$f_{n+1} = f_n + \frac{k}{z}\Delta\theta \qquad (2).$$

In the formula, $f_{n+1}$ is the operating frequency after adjustment (reduced operating frequency); $f_n$ is the operating frequency before adjustment; $z$ is the impedance; $k$ is a coefficient factor, and the specific value of $k$ can be set according to needs and is not limited here; $\Delta\theta$ is the difference between the phase difference of voltage and current and the target phase difference, and $\frac{k}{z}\Delta\theta$ is the obtained frequency adjustment amount $\Delta f$. Because $\Delta\theta$ is a negative value at this time, $\frac{k}{z}\Delta\theta$ is a negative value, and therefore the operating frequency after adjustment is reduced relative to the operating frequency before adjustment.

As shown in FIG 4, the step S4 includes:

S41, determining whether the phase difference change rate is less than zero;

S42, if yes, that is, if it is determined that the phase difference change rate is less than zero, increasing the operating frequency and calculating the adjusted operating frequency;

S43, if not, that is, if it is determined that the phase difference change rate is not less than zero, judging whether the phase difference change rate is within a preset range;

S44, increasing the operating frequency and calculating the adjusted operating frequency when it is determined to be within the preset range;

S45, reducing the operating frequency and calculating the adjusted operating frequency when it is determined that the frequency is not within the preset range.

In the step S43, the preset range is $K_\theta \pm \varepsilon$, wherein $K_\theta$ is mainly determined by the matching capacitor and the matching inductor, and can be easily obtained by calculating the slope value of the experimental sweep frequency curve, for example, it can be $2.5*10^{-4}$; and $\varepsilon$ is a set adjustment margin, which can be set as needed, for example, $K_\theta$ is 5%, which is not specifically limited here.

In the steps S42 and S44, when calculating the adjusted operating frequency, the formula (2) in step S2 can be used for calculation. However, at this time, since $\Delta\theta$ is a positive value, $\frac{k}{z}\Delta\theta$ is a positive value, that is, the frequency adjustment amount $\Delta f$ is a positive value, so that the adjusted operating frequency increases relative to the operating frequency before adjustment.

The inventors have found that the absolute value of the phase difference change rate can represent the size of the phase-locking coefficient just like the impedance. The larger the absolute value of the phase difference change rate, the smaller the phase-locking coefficient, and the smaller the operating frequency that needs to be adjusted for the same phase difference, and vice versa. Therefore, in another embodiment, the adjusted operating frequency can also be calculated in combination with phase difference change rate, that is, when calculating the adjusted operating frequency, the calculation is performed according to formula (3) as follows:

$$f_{n+1} = f_n + \frac{l}{\omega}\Delta\theta \qquad (3).$$

In the formula, $f_{n+1}$ is the operating frequency after adjustment (increased operating frequency); $f_n$ is the operating frequency before adjustment, $\omega$ is the absolute value of the phase difference change rate; $l$ is a proportionality factor, which can be set as needed and is not limited here; $\Delta\theta$ is the difference between the phase difference of voltage and current and the target phase difference, and at this time $\frac{l}{\omega}\Delta\theta$ is the obtained frequency adjustment amount $\Delta f$. Because $\frac{l}{\omega}$, $\Delta\theta$ are all positive values, the obtained frequency adjustment amount $\Delta f$ is a positive value, and the final adjusted operating frequency increases relative to the operating frequency before adjustment.

In the step S45, when calculating the adjusted operating frequency, in one embodiment, the calculation may be performed according to formula (4) as follows:

$$f_{n+1} = f_n - \frac{k}{z}\Delta\theta \qquad (4).$$

In the formula, $f_{n+1}$ is the operating frequency after adjustment (reduced operating frequency); $f_n$ is the operating frequency before adjustment; $z$ is the impedance; $k$ is a coefficient factor, and $\Delta\theta$ is the difference between the phase difference of voltage and current and the target phase difference. Because $\Delta\theta$ is a positive value, and $\frac{k}{z}\Delta\theta$ is the obtained frequency adjustment amount $\Delta f$, which is a positive value, so that the adjusted operating frequency is reduced relative to the operating frequency before adjustment.

Of course, in other embodiments, the phase difference change rate may also be used to replace the variable frequency coefficient for calculation, that is, when calculating the adjusted operating frequency, it may also be calculated according to formula (5) as follows:

$$f_{n+1} = f_n - \frac{l}{\omega}\Delta\theta \qquad (5).$$

In the formula, $f_{n+1}$ is the operating frequency after adjustment; $f_n$ is the operating frequency before adjustment; $\omega$ is the absolute value of the phase difference change rate; $l$ is a proportionality factor, which can be set according to needs; and $\Delta\theta$ is the difference between the phase difference of voltage and current and the target phase difference. At this time, $\frac{l}{\omega}\Delta\theta$ is the obtained frequency adjustment amount $\Delta f$. Because $\frac{l}{\omega}$, $\Delta\theta$ are all positive values, the obtained frequency adjustment amount $\Delta f$ is a positive value, so that the working frequency after adjustment is reduced relative to the working frequency before adjustment.

Example 2

This embodiment discloses an operating frequency tracking system for ultrasonic transducer, including:

A phase difference determination unit, configured to calculate a phase difference of voltage and current according to acquired voltage and current signals;
A judgment unit, configured to determine whether the phase difference of voltage and current is greater than a target phase difference;
A first adjustment unit, configured to reduce the operating frequency and calculate the adjusted operating frequency when the phase difference of voltage and current is not greater than the target phase difference; and
A second adjustment unit, configured to calculate a phase difference change rate, determine whether to increase or decrease the operating frequency according to the phase difference change rate, and calculate the adjusted operating frequency, when the phase difference of voltage and current is greater than the target phase difference.

Example 3

This embodiment discloses an ultrasonic device, including a processor and a memory, wherein the memory stores a program that can be called by the processor; wherein when the processor executes the program, the tracking method described in the above example 1 is implemented. The ultrasonic device may be an ultrasonic welding device or an ultrasonic surgical tool.

There are many implementation methods of the present disclosure, and all solutions formed by equivalent replacement or equivalent transformation fall within the scope of the present disclosure.

**Claims**

1. A frequency tracking method for ultrasonic transducer, comprising the following steps:

   S1, calculating a phase difference between voltage and current according to acquired voltage and current signals;
   S2, determining whether the phase difference of voltage and current is greater than a target phase difference;
   S3, reducing the operating frequency and calculating the adjusted operating frequency when it is determined that the phase difference of voltage and current is not greater than the target phase difference;
   S4, calculating a phase difference change rate, determining whether to increase or decrease the operating frequency according to the phase difference change rate, and calculating the adjusted operating frequency, when it is determined that the phase difference of voltage and current is greater than the target phase difference.

2. The tracking method according to claim 1, wherein the phase difference change rate is calculated according to formula (1) as follows:

$$R_P = (P_n - P_{n-1}) \div (f_n - f_{n-1}) \qquad (1),$$

   wherein $R_P$ is the phase difference change rate; $P_n$ is the phase difference of voltage and current calculated this time; $P_{n-1}$ is the phase difference of voltage and current calculated last time; $f_n$ is the operating frequency corresponding to the phase difference of voltage and current calculated this time; and $f_{n-1}$ is the operating frequency corresponding to the phase difference of voltage and current calculated last time.

3. The tracking method according to claim 1, wherein when calculating the adjusted operating frequency in the step S3, the calculation is performed according to formula (2) as follows:

$$f_{n+1} = f_n + \frac{k}{z}\Delta\theta \qquad (2),$$

   wherein, $f_{n+1}$ is the operating frequency after adjustment; $f_n$ is the operating frequency before adjustment; z is an impedance; k is a coefficient factor; and $\Delta\theta$ is the difference between the phase difference of voltage and current and the target phase difference.

4. The tracking method according to any one of claims 1 to 3, wherein determining whether to increase or decrease the operating frequency according to the phase difference change rate, and calculating the adjusted operating frequency in the step S4 comprises:

   S41, determining whether the phase difference change rate is less than zero;
   S42, increasing the operating frequency and calculating the adjusted operating frequency when it is determined that the phase difference change rate is less than zero;
   S43, determining whether the phase difference change rate is within a preset range when it is determined that the phase difference change rate is not less than zero;
   S44, increasing the operating frequency and calculating the adjusted operating frequency when it is determined that the phase difference change rate is within the preset range; and
   S45, reducing the operating frequency and calculating the adjusted operating frequency when it is determined that the phase difference change rate is not within the preset range.

5. The tracking method according to claim 4, wherein the preset range is $K_\theta \pm \varepsilon$, wherein $K_\theta$ can be obtained by calculating the slope value of an experimental sweep frequency curve, and $\varepsilon$ is a set adjustment margin.

6. The tracking method according to claim 4, wherein when calculating the adjusted operating frequency in the steps S42 and/or S44, the calculation is performed according to formula (3) as follows:

$$f_{n+1} = f_n + \frac{l}{\omega}\Delta\theta \qquad (3),$$

wherein $f_{n+1}$ is the operating frequency after adjustment; $f_n$ is the operating frequency before adjustment; $\omega$ is an absolute value of the phase difference change rate; $l$ is a proportionality factor; and $\Delta\theta$ is the difference between the phase difference of voltage and current and the target phase difference.

7. The tracking method according to claim 4, wherein when calculating the adjusted operating frequency in step S45, the calculation is performed according to formula (4) as follows:

$$f_{n+1} = f_n - \frac{k}{z}\Delta\theta \qquad (4),$$

wherein $f_{n+1}$ is the operating frequency after adjustment; $f_n$ is the operating frequency before adjustment; z is the impedance; $k$ is a coefficient factor; and $\Delta\theta$ is the difference between the phase difference of voltage and current and the target phase difference.

8. The tracking method according to claim 4, wherein when calculating the adjusted operating frequency in the step S45, the calculation is performed according to formula (5) as follows:

$$f_{n+1} = f_n - \frac{l}{\omega}\Delta\theta \qquad (5),$$

wherein $f_{n+1}$ is the operating frequency after adjustment; $f_n$ is the operating frequency before adjustment; $\omega$ is the absolute value of the phase difference change rate; $l$ is the proportionality factor; and $\Delta\theta$ is the difference between the phase difference of voltage and current and the target phase difference.

9. An operating frequency tracking system for ultrasonic transducer, comprising:

a phase difference determination unit, configured to calculate a phase difference of voltage and current according to acquired voltage and current signals;
a judgment unit, configured to determine whether the phase difference of voltage and the current is greater than a target phase difference;
a first adjustment unit, configured to reduce the operating frequency and calculate the adjusted operating frequency when it is determined that the phase difference of voltage and current is not greater than the target phase difference; and
a second adjustment unit, configured to calculate a phase difference change rate, determine whether to increase or decrease the operating frequency according to the phase difference change rate, and calculate the adjusted operating frequency, when it is determined that the phase difference of voltage and the current is greater than the target phase difference.

10. An ultrasonic device, comprising a processor and a memory, wherein the memory stores a program that can be called by the processor; and wherein when the processor executes the program, the tracking method as described in any one of claims 1 to 8 is implemented.

FIG 1

FIG 2

Calculating a phase difference of voltage
and current according to acquired
voltage and current signals

The phase
difference of voltage and
current is greater than target
phase difference

YES

NO

Calculating a phase difference change rate,
determining whether to increase or decrease
the operating frequency according to the
phase difference change rate, and calculating
the adjusted operating frequency

Reducing the operating frequency and
calculating the adjusted operating
frequency

FIG 3

Calculating a phase difference of voltage
and current according to acquired voltage
and current signals

The phase difference
of voltage and current is greater
than target phase
difference

YES

NO

Calculating a phase
difference change rate

Calculating the adjusted operating
frequency according to
$f_{n+1} = f_n + \triangle f$

The phase
difference change rate
is less than
zero

YES

NO

The phase
difference change rate
is within the range of
$K_\theta \pm \varepsilon$

YES

NO

Calculating the adjusted operating
frequency according to
$f_{n+1} = f_n - \triangle f$

FIG 4

| | INTERNATIONAL SEARCH REPORT | International application No.<br>**PCT/CN2022/124733** |
|---|---|---|

**A.    CLASSIFICATION OF SUBJECT MATTER**

A61B17/32(2006.01)i;B06B3/00(2006.01)i

According to International Patent Classification (IPC) or to both national classification and IPC

**B.    FIELDS SEARCHED**

Minimum documentation searched (classification system followed by classification symbols)

A61B17,B06B3

Documentation searched other than minimum documentation to the extent that such documents are included in the fields searched

Electronic data base consulted during the international search (name of data base and, where practicable, search terms used)

CNABS, SIPOABS, DWPI: 超声波换能器, 相位差, 频率, 变化率, 判断, 调整, change, difference, frequency, phase, rate, ultrasonic, transducer, adjust+, modify+, jug+,

**C.    DOCUMENTS CONSIDERED TO BE RELEVANT**

| Category* | Citation of document, with indication, where appropriate, of the relevant passages | Relevant to claim No. |
|---|---|---|
| PX | CN 114924497 A (INNOLCON MEDICAL TECHNOLOGY (SUZHOU) CO., LTD.) 19 August 2022 (2022-08-19)<br>    claims 1-10 | 1-10 |
| A | CN 108478253 A (INNOLCON MEDICAL TECHNOLOGY (SUZHOU) CO., LTD.) 04 September 2018 (2018-09-04)<br>    entire document | 1-10 |
| A | CN 111596544 A (SICHUAN UNIVERSITY) 28 August 2020 (2020-08-28)<br>    entire document | 1-10 |
| A | CN 113289880 A (TSINGHUA SHENZHEN INTERNATIONAL GRADUATE SCHOOL) 24 August 2021 (2021-08-24)<br>    entire document | 1-10 |
| A | JP 2001258089 A (OLYMPUS OPTICAL CORPORATION) 21 September 2001 (2001-09-21)<br>    entire document | 1-10 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **03 February 2023** | **08 February 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District,**<br>**Beijing 100088** | |
| Facsimile No. **(86-10)62019451** | Telephone No. |

Form PCT/ISA/210 (second sheet) (July 2022)

**INTERNATIONAL SEARCH REPORT**
Information on patent family members

International application No.

**PCT/CN2022/124733**

| Patent document cited in search report | | | Publication date (day/month/year) | Patent family member(s) | | | Publication date (day/month/year) |
|---|---|---|---|---|---|---|---|
| CN | 114924497 | A | 19 August 2022 | None | | | |
| CN | 108478253 | A | 04 September 2018 | EP | 3662850 | A1 | 10 June 2020 |
| | | | | EP | 3662850 | A4 | 02 June 2021 |
| | | | | WO | 2019024637 | A1 | 07 February 2019 |
| | | | | US | 2020297375 | A1 | 24 September 2020 |
| CN | 111596544 | A | 28 August 2020 | None | | | |
| CN | 113289880 | A | 24 August 2021 | None | | | |
| JP | 2001258089 | A | 21 September 2001 | JP | 3696034 | B2 | 14 September 2005 |

Form PCT/ISA/210 (patent family annex) (July 2022)

**EP 4 520 277 A1**

**Patent documents cited in the description**

- CN 201810159303